**Europäisches Patentamt**

**European Patent Office**

**Office européen des brevets**

(19)

(11) Veröffentlichungsnummer : **0 110 239
B1**

(12)   # EUROPÄISCHE PATENTSCHRIFT

(45) Veröffentlichungstag der Patentschrift :
26.03.86

(21) Anmeldenummer : 83111461.6

(22) Anmeldetag : 17.11.83

(51) Int. Cl.⁴ : **C 07 D209/88**

(54) **Verfahren zur Herstellung von 2-Hydroxy-carbazol-1-carbonsäure.**

(30) Priorität : 27.11.82 DE 3244024
16.04.83 DE 3313906

(43) Veröffentlichungstag der Anmeldung :
13.06.84 Patentblatt 84/24

(45) Bekanntmachung des Hinweises auf die Patenterteilung : 26.03.86 Patentblatt 86/13

(84) Benannte Vertragsstaaten :
CH DE GB LI NL

(56) Entgegenhaltungen :
BE-A-    357 270
US-A- 2 453 105
US-A- 3 655 697
US-A- 3 674 875
US-A- 3 759 948

(73) Patentinhaber : BAYER AG
Konzernverwaltung RP Patentabteilung
D-5090 Leverkusen 1 Bayerwerk (DE)

(72) Erfinder : Köhler, Wilfried, Dr.
Gustav-Freytag-Strasse 2
D-5090 Leverkusen.1 (DE)

## Beschreibung

Es ist bereits bekannt, 2-Hydroxy-carbazol in Gegenwart von Kaliumcarbonat bei 275 °C und 25 bar $CO_2$-Druck in einer 20-stündigen Reaktion zu einem Gemisch der isomeren 2-Hydroxy-carbazol-ortho-carbonsäuren umzusetzen (DE-PS 512 234). Ausbeuten und Selektivitäten sind hierbei nicht angegeben. In dieser DE-PS wird auch die Verwendung von Wasser als Reaktionsmedium beschrieben; die Nacharbeitung dieser Vorschrift ergab jedoch, daß der Umsatz dabei auf einen Bruchteil des Umsatzes in Abwesenheit von Wasser absinkt.

Unterwirft man 2-Hydroxy-carbazol der Kolbe-Schmitt-Reaktion, wird ebenfalls ein Gemisch der isomeren 2-Hydroxy-carbazol-ortho-carbonsäuren erhalten. Gemäß FIAT Final Report Nr. 1313, Seite 364 wird 2-Hydroxycarbazol mit wäßriger Kalilauge eingedampft und das so erhaltene trockene 2-Hydroxy-carbazol-kaliumsalz ab 180 °C und ansteigend bis 250-260 °C und 4,5 bar $CO_2$-Druck umgesetzt. Das Reaktionsgemisch wird in Wasser gelöst und mit Schwefelsäure neutralisiert, wobei das nicht umgesetzte 2-Hydroxy-carbazol ausfällt. Nach Aussalzen mit Steinsalz wird die 2-Hydroxy-carbazol-1-carbonsäure in Form ihres Natriumsalzes erhalten. Die isomere 3-Carbonsäure und Teile der 1-Carbonsäure gelangen ins Filtrat.

Aus US 2 453 105 ist die Carboxylierung von in o-Dichlorbenzol suspendiertem 2-Hydroxy-carbazol-kaliumsalz bekannt. Die Bildung des 2-Hydroxy-carbazol-kaliumsalzes aus 2-Hydroxy-carbazol und Kaliumhydroxid, die Entfernung des dabei entstehenden Wassers und die weitere Umsetzung in heterogener Phase mit $CO_2$ wird in Gegenwart von o-Dichlorbenzol als Dispergiermittel vorgenommen. Nach nicht genannter Reaktionsdauer wird das o-Dichlorbenzol mit Wasserdampf abdestilliert, durch Neutralisation mit Schwefelsäure das nicht umgesetzte 2-Hydroxycarbazol ausgefällt und durch Aussalzen mit Kaliumcarbonat das Kaliumsalz der 2-Hydroxy-carbazol-1-carbonsäure gewonnen. Die 3-Carbonsäure befindet sich sodann mit Resten der 1-Carbonsäure im Filtrat.

Auch in JP 5084/1956 wird die Carboxylierung von suspendiertem Kaliumsalz des 2-Hydroxycarbazols in 2-Chlornaphthalin beschrieben. Das aus Kaliumhydroxid hergestellte Salz wird zunächt entwässert und dann in Gegenwart einer katalytischen Menge Triethanolamin bei 170 bis 175 °C und 10 bar $CO_2$-Druck carboxyliert. Die Entfernung des nicht umgesetzten 2-Hydroxycarbazols und das Aussalzen und Gewinnen der isomeren Carbonsäuren geschieht wie in dem obenerwähnten US-Patent.

Alle bisherigen Verfahren leiden an den vielfältigen und umständlichen Trennoperationen und an der mangelnden Selektivität in Bezug auf das gewünschte Isomere.

Es wurde nun ein Verfahren zur Herstellung von 2-Hydroxycarbazol-1-carbonsäure durch Umsetzung von 2-Hydroxycarbazol-Alkalisalz mit $CO_2$ bei höherer Temperatur und unter Druck gefunden, das dadurch gekennzeichnet ist, daß man die Umsetzung in alkoholischen Lösungsmitteln unter Ausschluß von Wasser durchführt.

Lösungsmittel für das erfindungsgemäße Verfahren sind Alkohole und Etheralkohole.

Als Alkohole kommen sowohl 1-wertige als auch mehrwertige Alkohole in Betracht. Als 1-wertige Alkohole seien beispielsweise aliphatische, geradkettige oder verzweigte $C_1$-$C_8$-Alkohole genannt, wie Methanol, Ethanol, Propanol, Isopropanol, Butanol, Isobutanol, tert.-Butanol, Pentanole, Hexanole oder Octanole. Als mehrwertige Alkohole seien beispielsweise Glykol, 1,2- und 1,3-Dihydroxypropan genannt.

Etheralkohole als Lösungsmittel für das erfindungsgemäße Verfahren sind beispielsweise Verbindungen wie Glykolmonomethylether, Glykolmonoethylether und die sich vom Diglykol und Triglykol ableitenden Monoalkylether.

Alle genannten Lösungsmittel können sowohl einzeln als auch im Gemisch eingesetzt werden.

Unter den genannten Lösungsmitteln sind die 1-wertigen Alkohole, insbesondere die 1-wertigen $C_3$-$C_5$-Alkohole bevorzugt.

Als Kation für die Bildung des 2-Hydroxy-carbazolalkalisalzes seien Natrium und Kalium, insbesondere Kalium genannt. Zur Bildung des 2-Hydroxy-carbazolalkalisalzes kann beispielsweise das jeweilige Alkalimetall-hydroxid, -carbonat, -hydrogencarbonat oder -alkoholat eingesetzt werden. Beim Einsatz des Alkoholats kann beispielsweise von dem Alkohol ausgegangen werden, der auch als Lösungsmittel für das erfindungsgemäße Verfahren dient. Da jedoch auch Lösungsmittelgemische eingesetzt werden können, kann auch von einem anderen Alkoholat als dem des Lösungsmittel-Alkohols ausgegangen werden. Im allgemeinen ist es jedoch wegen der guten Zugänglichkeit bevorzugt, das Hydroxid des gewählten Metalls einzusetzen.

Das erfindungsgemäße Verfahren kann im Temperaturbereich von 80 bis 230 °C, bevorzugt 100 bis 180 °C, besonders bevorzugt 110 bis 170 °C durchgeführt werden.

Der $CO_2$-Druck für das erfindungsgemäße Verfahren kann in einem sehr großen Bereich schwanken, der lediglich durch die technischwirtschaftliche Handhabung begrent ist, beispielsweise von 0,5 bis 200 bar. Zur Durchführung wird man jedoch im allgemeinen einen Bereich von 1 bis 100 bar, bevorzugt 1,5 bis 40 bar, besonders bevorzugt 2 bis 20 bar und ganz besonders bevorzugt 3 bis 10 bar anwenden. Zusätzlich kann gegebenenfalls ein Inertgasdruck, beispielsweise bis zu 20 bar, angewendet werden. Inertgase sind beispielsweise Stickstoff, Wasserstoff oder Edelgase. Zu den genannten Drukken kommt in jedem Falle zusätzlich der Lösungsmitteldruck entsprechend dem Eigendruck bei der gewählten Reaktionstemperatur.

Das 2-Hydroxy-carbazol-salz wird für das erfindungsgemäße Verfahren in einer Konzentration von 5 bis 50 Gew.-%, bevorzugt 10 bis 40 Gew.-%, besonders bevorzugt 15 bis 30 Gew.-%, bezogen auf die Gesamtmenge Salz und Lösungsmittel, eingesetzt. Dabei herrscht bei Reaktionstemperatur eine Konzentration von gelöstem 2-Hydroxycarbazol-salz von mindestens 5 Gew.-%, meistens mehr als 15 Gew.-%, bezogen auf die Gesamtmenge an gelöstem 2-Hydroxycarbazol-salz und Lösungsmittel.

Zur Bildung des 2-Hydroxy-carbazol-salzes werden im allgemeinen 1 bis 2,5 Mol Alkali, bevorzugt 1-1,5 Mol, pro 1 Mol 2-Hydroxycarbazol angewendet.

Das erfindungsgemäße Verfahren wird unter Ausschluß von Wasser durchgeführt. Falls man nicht das 2-Hydroxy-carbazol-salz getrennt herstellt, trocknet und so bereits wasserfrei in das erfindungsgemäße Verfahren einsetzt, kann man es in dem gewählten Lösungsmittel oder Lösungsmittelgemisch auch aus 2-Hydroxycarbazol und einer der genannten Verbindungen des gewünschten Kations herstellen und das gegebenenfalls dabei entstehende Wasser vor der Carboxylierung aus dem Reaktionsgemisch abdestillieren. Dieses Abdestillieren ist eine allgemein bekannte Technik und kann durch Abdestillieren einer reinen Wasserphase oder durch Abdestillieren eines Wasser/Lösungsmittel-Gemisches, das gegebenenfalls ein Azeotrop sein kann, erfolgen.

Das erfindungsgemäße Verfahren kann beispielsweise so durchgeführt werden, daß der Reaktionsansatz nach der Bildung des 2-Hydroxy-carbazol-salzes so lange erhitzt wird, bis das gesamte vorhandene Wasser entfernt ist, wonach dann unter Einstellen des gewünschten $CO_2$-Druckes unter Rühren auf die gewünschte Reaktionstemperatur erhitzt wird. Die Reaktionszeiten liegen gewöhnlich zwischen 1 und 20 Stunden. Nach dem Abkühlen und Entspannen des Reaktionsansatzes liegt das 2-Hydroxycarbazol-1-carbonsäuresalz als aus dem Reaktionsgemisch ausgefallener Niederschlag vor. Es kann durch geeignete Trennoperationen, wie Abfiltrieren oder Abzentrifugieren, gewonnen werden. Das Filtrat enthält sodann nur geringe Mengen an gelöstem Carbonsäure-salz sowie nicht umgesetztes 2-Hydroxy-carbazol-salz. In einer bevorzugten Variante wird dieses Filtrat bei einem folgenden Reaktionsansatz wiederverwendet.

Das erfindungsgemäße Verfahren erlaubt die direkte Gewinnung einer 2-Hydroxy-carbazol-1-carbonsäure mit nur sehr geringem Gehalt an der nicht gewünschten isomeren 3-Carbonsäure und an nicht umgesetztem 2-Hydroxy-carbazol. Das erfindungsgemäße Verfahren vermeidet daher eine nachträgliche Abtrennung der unerwünschten 3-Carbonsäure. Hierzu ist es insbesondere nicht notwendig, eine Trennung durch Aussalzen, wie aus den bisher bekannten Verfahren geläufig, anzuwenden, wodurch der Aufwand an zum Aussalzen benötigten Chemikalien und die Behandlung hoher Salzfrachten in den Abwässern vermieden werden. Weiterhin ist es erfindungsgemäß nicht notwendig, eine gesonderte Trennoperation für die Abtrennung des nicht umgesetzten 2-Hydroxy-carbazol-salzes durchzuführen. Die 2-Hydroxy-carbazol-1-carbonsäure fällt im erfindungsgemäßen Verfahren zunächst als Salz des gewählten Kations an und kann als solches direkt weiterverwendet werden, beispielsweise zur Herstellung von Naphthol-AS-Farbstoffen. Die freie Säure kann in bekannter Art durch Ansäuern, beispielsweise mit Hilfe einer Mineralsäure, gewonnen werden.

Beispiel 1

In eine gerührte Suspension von 239 g technischem 2-Hydroxycarbazol (94 %ig) in 1 250 ml iso-Butanol ließ man bei ca. 80 °C 561 g einer 25,7 gew.-%igen Lösung von Kalium-iso-butanolat in iso-Butanol einfließen. Man entfernte Wasser-Reste durch Abdestillieren von ca. 550 ml iso-Butanol. Das in Bezug auf das Salz ca. 20 Gew.-%ige Reaktionsgemisch rührte man im Autoklaven bei 150 °C unter einem $CO_2$-Druck von 2,5 bar 10 Std. lang. Der Autoklav wurde abgekühlt, entleert, mit iso-Butanol nachgespült, und bei 80-90 °C wurde das ungelöste 2-Hydroxycarbazol-1-carbonsäure-kaliumsalz abfiltriert. Nach Waschen mit 80 °C heißem iso-Butanol und Trocknen im Vakuum wurden 295 g Kristalle erhalten.

Analyse

2-Hydroxycarbazol-1-carbonsäure-kaliumsalz : 98,3 Gew.-%
2-Hydroxycarbazol-3-carbonsäure-kaliumsalz : 1,5 Gew.-%
2-Hydroxycarbazol : nicht nachweisbar.

Ausbeute

1-Carbonsäure : 89 % d.Th. bez. auf Einsatz 2-Hydroxy-carbazol :
98 % d. Th. bez. auf umgesetztes 2-Hydroxycarbazol.

Im Filtrat sind enthalten, bezogen auf Einsatz 2-Hydroxycarbazol :
1-Carbonsäure : 0,9 % d.Th.
3-Carbonsäure : 0,1 % d.Th.
2-Hydroxycarbazol : 8,6 % vom Einsatz.

Beispiel 2

In eine gerührte Suspension von 241 g technischem 2-Hydroxycarbazol (93 %ig) in 1 600 ml n-Butanol ließ man bei 90 °C 157 g 48 %ige wäßrige Kalilauge einlaufen. Man erhitzte in einer Destillationsapparatur, versehen mit einer 70-cm-Füllkörperkolonne und einem auf die Kolonne aufgesetzten Wasserabscheider unter Rückfluß, bis sich kein Wasser mehr abschied. Anschließend wurden noch 250 ml n-Butanol abdestilliert, um letzte Wasserreste zu entfernen. Das Reaktionsgemisch wurde im Autoklaven bei 150 °C unter einem $CO_2$-Druck von 2,5 bar 10 Std. lang gerührt. Die Aufarbeitung erfolgte, wie in Beispiel 1 beschrieben ist. Es wurden 281 g Kristalle erhalten.

Analyse

2-Hydroxycarbazol-1-carbonsäure-kaliumsalz :
98,4 Gew.-%
2-Hydroxycarbazol-3-carbonsäure-kaliumsalz :
1,3 Gew.-%
2-Hydroxycarbazol : nicht nachweisbar

Ausbeute

1-Carbonsäure : 85 % d.Th. bez. auf Einsatz 2-Hydroxycarbazol ;
97 % d.Th. bez. auf umgesetztes 2-Hydroxycarbazol.

Im Filtrat sind enthalten, bezogen auf Einsatz 2-Hydroxycarbazol :
1-Carbonsäure : 1,2 % d.Th.
3-Carbonsäure : 0,2 % d.Th.
2-Hydroxycarbazol : 12,4 % vom Einsatz.

Beispiel 3

In eine gerührte Suspension von 239 g technischem 2-Hydroxycarbazol (94 %ig) in 1 250 ml n-Butanol ließ man bei ca. 80 °C 614 g einer 25 gew.-%igen Lösung von Kalium-n-butanolat in n-Butanol einfließen. Man entfernte Wasser-Reste durch Abdestillieren von ca. 600 ml n-Butanol. Carboxylierung und Aufarbeitung der Reaktionsmischung erfolgten, wie in Beispiel 1 beschrieben wurde ; Ausbeute 287 g Kristalle.

Das angefallene Filtrat wurde mit 215 g technischem 2-Hydroxycarbazol (94 %ig) versetzt. Man überführte mit 515 g einer 25 gew.-%igen Lösung von Kalium-n-butanolat in n-Butanol, wie oben beschrieben, in das 2-Hydroxycarbazol-kaliumsalz, destillierte n-Butanol ab bis zu einer Konzentration an 2-Hydroxycarbazol-kaliumsalz von ca. 20 Gew.-%, carboxylierte und arbeitete auf ; Ausbeute 294 g Kristalle.

Das hierbei angefallene Filtrat wurde mit der gleichen Menge an 2-Hydroxycarbazol versetzt wie bei der ersten Rückführung. Überführung in das Kaliumsalz und Carboxylierung erfolgten entsprechend :
Ausbeute 294 g Kristalle.
Gesamtausbeute : 875 g Kristalle.

Analyse der Gesamt-Kristallmischung

2-Hydroxycarbazol-1-carbonsäure-kaliumsalz :
98.0 Gew.-%
2-Hydroxycarbazol-3-carbonsäure-kaliumsalz :
1,5 Gew.-%
2-Hydroxycarbazol : nicht nachweisbar

Ausbeute

1-Carbonsäure : 94 % d.Th., bezogen auf insgesamt eingesetztes 2-Hydroxycarbazol.

Im letzten Filtrat sind enthalten, bezogen auf Gesamteinsatz 2-Hydroxycarbazol :

1-Carbonsäure : 0,8 % d.Th.
2-Carbonsäure : 0,1 % d.Th.
2-Hydroxycarbazol : 3,6 % d.Th.

**Patentansprüche**

1. Verfahren zur Herstellung von 2-Hydroxy-carbazol-1-carbonsäure durch Umsetzung von 2-Hydroxy-carbazol-alkalisalz mit $CO_2$ bei höherer Temperatur und unter Druck, dadurch gekennzeichnet, daß man die Umsetzung in alkoholischen Lösungsmitteln unter Ausschluß von Wasser durchführt.

2. Verfahren nach Anspruch 1, dadurch gekennzeichnet, daß man das 2-Hydroxy-carbazol-kaliumsalz einsetzt.

3. Verfahren nach Anspruch 1 bis 2, dadurch gekennzeichnet, daß man einen 1-wertigen Alkohol als Lösungsmittel einsetzt.

4. Verfahren nach Anspruch 1 bis 2, dadurch gekennzeichnet, daß man einen 1-wertigen $C_3$-$C_5$-Alkohol einsetzt.

5. Verfahren nach Anspruch 1 bis 4, dadurch gekennzeichnet, daß man das 2-Hydroxy-carbazol-salz in einer Konzentration von 5 bis 50 Gew.-%, bezogen auf die Gesamtmenge des Salzes und des Lösungsmittels, einsetzt.

6. Verfahren nach Anspruch 1 bis 4, dadurch gekennzeichnet, daß man das 2-Hydroxy-carbazol-salz in einer Konzentration von 10 bis 40 Gew.-%, bezogen auf die Gesamtmenge Salz und Lösungsmittel, einsetzt.

7. Verfahren nach Anspruch 1 bis 6, dadurch gekennzeichnet, daß man die Umsetzung bei Temperaturen von 80 bis 230 °C durchführt.

8. Verfahren nach Anspruch 1 bis 7, dadurch gekennzeichnet, daß man nach dem Abtrennen des ausgefallenen Salzes der 2-Hydroxy-carbazol-1-carbonsäure das verbleibende Lösungsmittel mit den darin enthaltenen Mengen an nicht umgesetztem 2-Hydroxy-carbazol-salz in die Reaktion zurückführt.

**Claims**

1. Process for the preparation of 2-hydroxycarbazole-1-carboxylic acid by reaction of an alkali metal salt of 2-hydroxycarbazole with $CO_2$ at elevated temperature and under pressure, characterised in that the reaction is carried out in alcoholic solvents with the exclusion of water.

2. Process according to Claim 1, characterised in that the potassium salt of 2-hydroxycarbazole is employed.

3. Process according to Claim 1 to 2, characterised in that a monohydric alcohol is employed as the solvent.

4. Process according to Claim 1 to 2, characterised in that a monohydric $C_3$-$C_5$-alcohol is employed.

5. Process according to Claim 1 to 4, characterised in that the salt of 2-hydroxycarbazole is employed in a concentration of 5 to 50 % by weight relative to the total amount of the salt and the solvent.

6. Process according to Claim 1 to 4, characterised in that the salt of 2-hydroxycarbazole is employed in a concentration of 10 to 40 % by weight relative to the total amount of salt and solvent.

7. Process according to Claim 1 to 6, characterised in that the reaction is carried out at temperatures from 80 to 230 °C.

8. Process according to Claim 1 to 7, characterised in that, after removal of the precipitated salt of 2-hydroxycarbazole-1-carboxylic acid, the remaining solvent with the amounts of unreacted salt of 2-hydroxycarbazole contained therein in returned to the reaction.

**Revendications**

1. Procédé de production d'acide 2-hydroxy-carbazole-1-carboxylique par réaction d'un sel alcalin de 2-hydroxycarbazole avec $CO_2$ à température élevée et sous pression, caractérisé en ce qu'on conduit la réaction dans des solvants alcooliques en opérant en l'absence d'eau.

2. Procédé suivant la revendication 1, caractérisé en ce qu'on utilise le sel de potassium, du 2-hydroxycarbazole.

3. Procédé suivant les revendications 1 et 2, caractérisé en ce qu'on utilise un alcool monovalent comme solvant.

4. Procédé suivant les revendications 1 et 2, caractérisé en ce qu'on utilise un alcool monovalent en $C_3$ à $C_5$.

5. Procédé suivant les revendications 1 à 4, caractérisé en ce qu'on utilise le sel de 2-hydroxycarbazole à une concentration de 5 à 50 % en poids par rapport à la quantité totale du sel et du solvant.

6. Procédé suivant les revendications 1 à 4, caractérisé en ce qu'on utilise le sel de 2-hydroxycarbazole à une concentration de 10 à 40 % en poids par rapport à la quantité totale de sel et de solvant.

7. Procédé suivant les revendications 1 à 6, caractérisé en ce qu'on conduit la réaction à des températures de 80 à 230 °C.

8. Procédé suivant les revendications 1 à 7, caractérisé en ce qu'après la séparation du sel précipité de l'acide 2-hydroxycarbazole-1-carboxylique on recycle dans la réaction le solvant restant avec les quantités qui y sont contenues de sel de 2-hydroxycarbazole n'ayant pas réagi.